(19) **Europäisches Patentamt European Patent Office Office européen des brevets**

(11) **EP 1 670 934 B1**

(12) **EUROPEAN PATENT SPECIFICATION**

(45) Date of publication and mention of the grant of the patent:
**29.10.2008 Bulletin 2008/44**

(21) Application number: **04765756.4**

(22) Date of filing: **01.10.2004**

(51) Int Cl.:
*C12Q 1/02* (2006.01)  *A23L 1/308* (2006.01)

(86) International application number:
**PCT/EP2004/010997**

(87) International publication number:
**WO 2005/035781 (21.04.2005 Gazette 2005/16)**

(54) **PREBIOTIC EFFECT ANALYSIS**

PRÄBIOTISCHE EFFEKTANALYSE

ANALYSE D'EFFET PREBIOTIQUE

(84) Designated Contracting States:
**AT BE BG CH CY CZ DE DK EE ES FI FR GB GR HU IE IT LI LU MC NL PL PT RO SE SI SK TR**

(30) Priority: **02.10.2003 GB 0323089**
**28.01.2004 GB 0401867**

(43) Date of publication of application:
**21.06.2006 Bulletin 2006/25**

(73) Proprietor: **Nestec S.A.**
**1800 Vevey (CH)**

(72) Inventors:
- **VULEVIC, Jelena**
  **Reading RG1 6SW (GB)**
- **GIBSON, Glenn R.**
  **Reading, Berkshire RG2 9DW (GB)**
- **RASTALL, Robert**
  **Reading RG1 5PX (GB)**

(74) Representative: **Lock, Graham James et al**
**Fry Heath Spence**
**The Gables**
**Massetts Road**
**Horley, Surrey RH6 7DQ (GB)**

(56) References cited:
- **SMIRICKY-TJARDES M R ET AL: "Dietary galactooligosaccharides affect ileal and total-tract nutrient digestibility, ileal and fecal bacterial concentrations, and ileal fermentative characteristics of growing pigs." JOURNAL OF ANIMAL SCIENCE, vol. 81, no. 10, October 2003 (2003-10), pages 2535-2545, XP002313476 ISSN: 0021-8812**

- **PALFRAMAN R ET AL: "Development of a quantitative tool for the comparison of the prebiotic effect of dietary oligosaccharides." LETTERS IN APPLIED MICROBIOLOGY. 2003, vol. 37, no. 4, 2003, pages 281-284, XP002313465 ISSN: 0266-8254 cited in the application**
- **OLANO-MARTIN E ET AL: "Comparison of the in vitro bifidogenic properties of pectins and pectic-oligosaccharides" JOURNAL OF APPLIED MICROBIOLOGY, vol. 93, no. 3, 2002, pages 505-511, XP002313467 ISSN: 1364-5072 cited in the application**
- **FEMIA ANGELO PIETRO ET AL: "Antitumorigenic activity of the prebiotic inulin enriched with oligofructose in combination with the probiotics Lactobacillus rhamnosus and Bifidobacterium lactis on azoxymethane-induced colon carcinogenesis in rats." CARCINOGENESIS (OXFORD), vol. 23, no. 11, November 2002 (2002-11), pages 1953-1960, XP002314109 ISSN: 0143-3334**
- **VULEVIC JELENA ET AL: "Developing a quantitative approach for determining the in vitro prebiotic potential of dietary oligosaccharides" FEMS MICROBIOLOGY LETTERS, vol. 236, no. 1, July 2004 (2004-07), pages 153-159, XP002313418 ISSN: 0378-1097**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention concerns a method for providing a nutritional or pharmaceutical composition comprising a prebiotic to an individual in need thereof.

Dietary interventions for the treatment or prevention of diseases are highly sought after to relieve the burdens on medical service providers. One of the most promising current targets for such dietary interventions is the gastrointestinal tract. The large intestine is, by far, the most densely populated area of the gut. Its resident microflora appears to be associated with control of transit time, bowel habit, absorptive and mucosal function as well as implicated, through its normal metabolic state, in many other physiological processes relevant to host health and disease. Modulation of the composition of this microflora through use of certain food ingredients, offers an attractive basis for developing novel dietary therapies. This has resulted in the development of pro and prebiotic concepts. Both rely upon enhancement of beneficial components of the microflora, such as bifidobacteria and lactobacilli. Whilst the former concept uses a live microbial supplement, the latter is defined as a nondigestible food ingredient which selectively stimulates growth and/or activity of these beneficial groups of bacteria.

Health claims attributed to the consumption of probiotics have been reported for a long time. However, one of the main problems of this approach is survival of the probiotic in the complex gut ecosystem. Following ingestion, strains are confronted by factors such as gastric acids, bile salts and various enzymes as well as the ability to establish in the large intestine and compete with a diverse and metabolically active indigenous microbiota. An alternative to the survivability problems lies in the use of prebiotics and it may be assumed that these will be used as predominant ingredients in future developments of nutritional approaches for the treatment or prevention of diseases and disorders concerning the gastrointestinal tract.

Prebiotics are non-digestible food ingredients, e.g. non-digestible carbohydrates, which have a beneficial effect on the health. For a food ingredient to be classified as a prebiotic it must fulfill the following criteria: i) neither be hydrolyzed nor absorbed in the gastrointestinal tract, ii) be selectively fermented by one or a limited number of potentially beneficial bacteria commensal to the colon, such as lactobacilli and bifidobacteria, which are stimulated to grow and/or become metabolically activated, iii) be able to alter the colonic microflora towards a healthier composition, by increasing, for example, numbers of saccharolytic species while reducing putrefactive microorganisms. The fermentation of the prebiotic by the colonic bacteria may lead to the production of short chain fatty acid (SCFA), such as succinic, lactic, formic, acetic, propionic, isobutyric, butyric, isovaleric and valeric acids, hydrogen and carbon dioxide gases.

In recent years, there is on the part of the consumers an increasing demand for foodstuffs particular there is an interest in developing functional foods having prebiotic capability. *In vivo* human studies have shown that dietary addition of particular oligosaccharides, such as fructooligosaccharides (FOS), may lead to an increase in beneficial faecal bifidobacteria. Nevertheless, there is no uniform method to determine the effectiveness of different fibers to act as prebiotics and to compare their prebiotic capabilities.

There is a need to develop improved nutritional compositions containing prebiotics. To do so, a method that will allow quantification and/or comparison of the prebiotic capabilities of different fibers would be particularly useful.

In one aspect of the invention there is provided a comparative and standardized method, as herein defined as the method of the invention, for assessing the prebiotic capability of a dietary fiber, e.g. a carbohydrate, e.g. an oligosaccharide, in particular through its effect on faecal bacteria.

In a specific embodiment of the invention, the method of the invention allows the evaluation or quantification of the effect of the tested fiber on the growth of faecal bacteria, in particular of beneficial or potentially beneficial faecal bacteria, and/or on the modification of the faecal bacteria population, in particular towards a healthier composition.

In another embodiment of the invention, the method of the invention is a method for evaluating the fermentation of dietary carbohydrates, e.g. oligosaccharides, and optionally for comparing their prebiotic effect.

The method of the invention may compare measurements of bacterial changes, e.g. faecal bacterial changes, e.g. through the determination of i) amount, e.g. number, e.g. $log_{10}$ number, or growth rate, e.g. maximum growth rate, of the bacteria, and/or i) rate of substrate, e.g. carbohydrate, e.g. oligosaccharide, assimilation and/or iii) production of fermentation end products, e.g. short chain fatty acids, such as lactic, acetic, propionic and butyric acids.

In one embodiment of the invention, the method of the invention is a subtractive culture method.

As used herein, a "subtractive culture method" refers to a method which may include at least the following steps: 1)- incubating a faecal bacterial culture in parallel in the presence and in the absence of the tested fiber during a certain incubation period, e.g. until the substrate or the tested fiber is completely fermented; 2)- determining the amount, e.g. number, $log_{10}$ number, of the faecal bacteria in the culture, in the presence and in the absence of the tested fiber, in particular of beneficial or potentially beneficial faecal bacteria in the presence and in the absence of the tested fiber of non beneficial faecal bacteria in the same conditions; and 3)- comparing the amount, e.g. number, $log_{10}$ number, of faecal bacteria in the culture in the presence and in the absence of the tested fiber, in particular of beneficial or potentially beneficial faecal bacteria versus non beneficial faecal bacteria.

The step 3) of the subtractive culture method according to the invention may include the following sub-steps:

3.1) subtracting the amount, of the beneficial or potentially beneficial faecal bacteria in the culture in the absence of the tested fiber from the amount, e.g. number, of the beneficial or potentially beneficial faecal bacteria in the culture in the presence of the tested fiber; 3.2) subtracting the amount, e.g. number, of the non beneficial faecal bacteria in the culture in the absence of the tested fiber from the amount, e.g. number, of the non beneficial faecal bacteria in the culture in the presence of the tested fiber, and 3.3) subtracting the value as determined in step 3.2) from the value as determined in step 3.1) to get a value which may be reflective of the prebiotic capability of the tested fiber.

In another embodiment of the invention, the calculation of the amount of faecal bacteria may include, e.g. may consist in, calculating the growth rate, e.g. maximum growth rate of the bacterial population.

Figure 1 shows the concentration of residual substrate in stirred pH-controlled batch cultures as measured by total carbohydrate assay. ▲ 1% (w/v) sucrose, ♦ 1% (w/v) guar gum, ■ 1% (w/v) FOS and • 1% (w/v) tGOS.
Figure 2 shows the production of SCFA as determined by HPLC in an *in vitro* gut model models containing 1% (w/v) partially hydrolyzed guar gum (PHGG) as substrate.
Figure 3 shows the production of SCFA as determined by HPLC in an *in vitro* gut model models containing 1% (w/v) FOS as substrate.
Figure 4 shows the production of SCFA as determined by HPLC in an *in vitro* gut model models containing 1 % (w/v) tGOS as substrate.

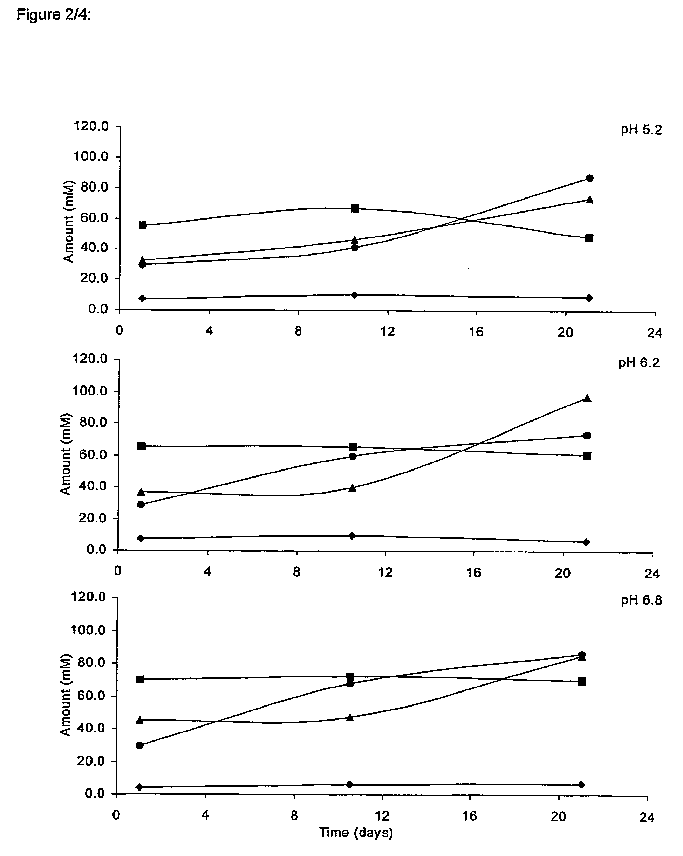

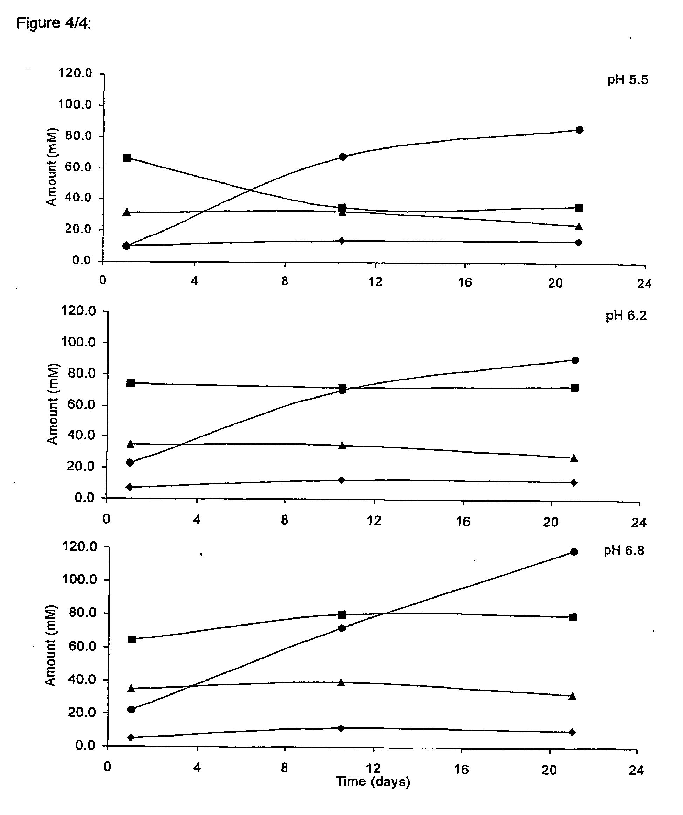

In the Figures 2, 3 and 4, the three vessels of the model correspond to pH 5.5, pH 6.2 and pH 6.8. ■ acetic, lactic, A propionic and • butyric acid.

As used herein, the "prebiotic capability of a fiber" refers to the capability of a fiber to act as a prebiotic, e.g. to be fermented by beneficial or potentially beneficial faecal bacteria, such as lactobacilli and bifidobacteria, and/or to be able to alter the faecal bacterial population towards a healthier composition, e.g. in stimulating the growth and/or the metabolism of beneficial or potentially beneficial faecal bacteria and/or in inhibiting the growth and/or the metabolism of non beneficial or pathogenic faecal bacteria.

According to the invention there is provided a method for evaluating or quantifying the prebiotic capability of a fiber or for identifying a prebiotic substance, which comprises (a) a step of evaluating or quantifying the stimulation by the tested fiber or substance on the growth and/or modification of a faecal bacterial population, (b) a step of quantifying at least one product resulting from the fermentation of the tested fiber or substance, (c) a step of quantifying the rate of assimilation of the tested fiber or substance, (d) a step of calculating the Measure of the Prebiotic Effect (MPE) using the following equation:

$$\frac{1}{2}\sqrt{x^2 y^2 + x^2 z^2 + y^2 z^2} = MPE$$

wherein x is the value as determined in step (a), *y* is the value as determined in step (b) and *z* is the value as determined in step (c), and (e) a step of evaluating or quantifying the probiotic capability of the tested fiber or substance as a function of the MPE.

A nutritional or pharmaceutical composition comprising at least one prebiotic may be used for treating and/or preventing GI disorders, such as diseases, conditions and symptoms related to chronic gut disorder, e.g. IBD, in particular ulcerative colitis, Crohn's disease, colon cancer or IBS or its syndromes in a mammal, including a human.

According to the invention, step (a) may comprise the step of quantifying the effect of the tested fiber, or blend of tested fibers, on faecal bacteria, e.g. on faecal bacterial growth and/or on changes in faecal bacterial population, e.g. on the multiplication and/or the metabolism of the faecal bacteria. Accordingly, step (a) may comprise the step of monitoring and/or quantifying the effect of the tested fiber, or blend of tested fibers, in stimulating the multiplication and/or activating the metabolism of beneficial or potentially beneficial faecal bacteria, such as e.g. lactobacilli and/or bifidobacteria, and/or in inhibiting the multiplication and/or the metabolism of non beneficial and/or detrimental faecal bacteria, such as e.g. saccharolytic and/or putrefactive species. Preferably, such a monitoring and/or quantifying analysis may comprise the calculation of the growth rates, e.g. the maximum growth rates, of feacal bacteria, e.g. of beneficial or potentially beneficial feacal bacteria and of non beneficial faecal bacteria.

According to the invention, such a monitoring and/or quantifying analysis can be done e.g. in anaerobic batch cultures containing the tested fiber, or blend of tested fibers, e.g. as sole carbon source, and inoculated by faecal bacteria, for instance in the form of faecal samples. The faecal samples may be obtained from a healthy human and/or a human who did not receive any antibiotic treatment for at least 6 months. Sucrose may be used as a control substrate as it is not selective, i.e. may be fermented by all bacteria. As a negative control culture medium without any substrate, e.g. without any fiber or any sugar, may be used.

In one aspect of the invention, step (a) may comprise a comparative and standardized method, e.g. a calculation method.

In one aspect of the invention, step (a) may comprise the step of comparing the effect of the tested fiber, or blend of tested fibers, on faecal bacteria to the effect of another fiber, e.g. a known prebiotic, e.g. FOS, to the same faecal bacteria in the same conditions.

In another aspect of the invention, step (a) may comprise a process for determining the "prebiotic index" (PI) of a fiber. The basic concept of PI is to derive a single number that quantifies the effect of the fiber on the bacterial, e.g. the faecal bacterial population, e.g. on the amount of beneficial faecal bacteria versus non beneficial faecal bacteria.

The prebiotic index may be calculated by i) summing increases of amounts, e.g. $\log_{10}$ populations, of beneficial and potentially beneficial faecal bacteria, e.g. bifidobacteria, lactobacilli and/or eubacteria and ii) adding the decrease or subtracting the increase to the numerical value obtained for the amounts, e.g. $\log_{10}$ populations, of non beneficial faecal bacteria, e.g. clostridia, bacteroides, coliforms and/or Sulfate Reducing bacteria, as determined e.g. in anaerobic fermentation culture, e.g. in a batch culture and/or a fermentation vessel.

According to the invention, the amounts, e.g. $\log_{10}$ populations, of faecal bacteria may be determined by dividing the numbers of bacteria at inoculation by the number of bacteria at sample time.

The following equation may be used to describe the prebiotic index:

$$PI = \Delta B + \Delta L + \Delta E - \Delta Ba - \Delta Cl - \Delta Co - \Delta SRB$$

wherein PI = prebiotic index; $\Delta$ = amount of bacteria, e.g. $\log_{10}$ bacterial populations, e.g. the number of bacteria at sample time divided by the number of bacteria at inoculation; B = bifidobacteria, e.g. *Bifidobacterium* spp; L = lactobacilli, e.g. *Lactobacillus* spp; E = eubacteria, e.g. *Eubacterium* rectale; Ba = bacteroides, e.g. *Bacteroides* spp ; Cl = clostridia, e.g. histolyticum group, e.g. *Clostridium* coccoides, *Clostridium* histolyticum, *Clostridium* difficile; Co = coliforms, e.g. *Escherichia* coli and SRB = Sulfate Reducing bacteria, e.g. *Desulfovibrio* spp.

In one embodiment of the invention, the amount of bacteria may correspond to the amount of bacteria, e.g. $\log_{10}$ bacterial populations, in presence of the fiber minus the amount of bacteria, e.g. $\log_{10}$ bacterial populations, in absence of the fiber.

In another embodiment of the invention, the prebiotic index may be described by the following equation:

$$PI = B/Total + L/Total + E/Total - Ba/Total - Cl/Total - Co/Total - SRB/Total$$

wherein "B", "L", "E", "Ba", "Cl", "Co" and "SRB" have the same meaning as above, and "Total" represents the total number of feacal bacteria at inoculation.

For example, the prebiotic index may be calculated as described in R. Palframan et al. (Letters in Applied Microbiology 2003, 37, p. 281 - 284), the content of which (in particular page 282 column 1, paragraph 2, to page 282, column 2, paragraph 1) is incorporated herein by reference, or as described in E. Olano-Martin et al. (Journal of Applied Microbiology 2002, 93, p. 505 - 511) the content of which (in particular page 508, last paragraph, to page 509, paragraph 2) is incorporated herein by reference.

In another embodiment of the invention, the prebiotic index of a fiber may be described through the growth rate, e.g. maximum growth rate, of the feacal bacteria, e.g. of the predominant beneficial feacal bacteria, versus the growth rate, e.g. maximum growth rate, of non beneficial feacal bacteria, e.g. in anaerobic batch cultures.

The growth rate of a bacteria population can be calculated with the following equation:

$$\ln N_t = \ln N_0 + \mu t$$

wherein

$N$ is the number of bacteria after the time interval, $t$, e.g. in hours,
$N_0$ is the initial number of bacteria and
$\mu$ is the specific growth rate, e.g. per hour.

In one aspect of the invention, the analysis of the faecal bacterial population, e.g. the measure of the PI, can be done though the growth rates calculated during the exponential phase of bacterial growth, i.e. the maximum growth

rates, i.e. $\mu_{max}$. The following equation may be used:

$$PI = \mu_{max} B + \mu_{max} L + \mu_{max} E - \mu_{max} Ba - \mu_{max} Cl - \mu_{max} Co - \mu_{max} SRB;$$

wherein PI = prebiotic index; $\mu_{max}$ = maximum growth rates of feacal bacteria in presence of the tested fiber, e.g. blend of tested fibers; B, L, E, Ba, Cl, Co, and SRB as hereinabove described.

The prebiotic index may be calculated by i) summing the growth rates, e.g. maximum growth rates, of the beneficial and potentially beneficial faecal bacteria, e.g. of bifidobacteria, lactobacilli and/or Eubacteria; ii) summing the growth rates, e.g. maximum growth rates, of the non beneficial faecal bacteria, e.g. of bacteroides, clostridia, coliforms, and/or Sulfate Reducing bacteria; and iii) subtracting the growth rate obtained under point ii) to the growth rate obtained under point i).

According to the invention, the Prebiotic Index may be weighted not to incorporate all the bacteria group in the same manner.

The Prebiotic Index may be expanded to take into account other bacteria group or species.

According to the invention, the Prebiotic Index may have a positive or negative value. The sign of the PI value (positive or negative) may be determined by the proportion of desired bacterial groups, e.g. bifidobacteria, lactobacilli and/or eubacteria, versus less desirable bacterial groups, e.g. clostridia, coliforms, bacteroides and/or sulftate reducing bacteria.

The extent of the Prebiotic Index value may be determined by the selectivity of the substrate, e.g. of the oligosaccharide.

In one embodiment of the invention, a fiber which gives a Prebiotic Index, as hereinabove described, greater than 0, greater than 0.1, 0.2, 0.3, 0.5 or greater than 1 may be considered as a good or suitable prebiotic.

According to the invention, step (b) may comprise the step of quantifying the capacity of the tested fiber, or blend of tested fiber, to be fermented by the faecal bacteria, e.g. by the beneficial and potentially beneficial faecal bacteria. The capacity of the tested fiber to be fermented may be monitoring or quantifying e.g. through i) the production of fermentation end products, such as short chain fatty acids (SCFA), e.g. lactic acid, propionic acid, acetic acid, and/or butyric acids, and/or ii) the rate of substrate assimilation, e.g. the rate of the fiber breakdown, and/or iii) the fermentation time, i.e. the time necessary for the fiber to be fermented, e.g. completely fermented, e.g. by the beneficial and potentially beneficial faecal bacteria.

As used herein, the term "substrate" refers to any molecule, e.g. carbohydrate, e.g. fiber, which can be assimilated by the feacal bacteria, e.g. when included in a medium culture, e.g. a batch culture or a fermentation vessel.

In one aspect of the invention, step (d) according to the invention comprises a process for determining the "measure of the prebiotic effect" (MPE). The basic concept of MPE is to derive a single number that quantifies the prebiotic capability of the fiber, e.g. its effect on the faecal bacterial, e.g. on the faecal bacterial growth and/or faecal bacterial population changes, e.g. as determined by the PI, and/or represents its capacity to be fermented by the faecal bacteria, e.g. to induce the production of fermentation end products, such as short chain fatty acids (SCFA). The prebiotic capability of the fiber, e.g. the MPE, may be evaluated, e.g. quantified, by monitoring the production of fermentation end products, such as short chain fatty acids (SCFA). The prebiotic capability of the fiber, e.g. the MPE, may also be evaluated, e.g. quantified, by analyzing, e.g. quantifying the rate of substrate assimilation, e.g. of fiber breakdown. The prebiotic capability of the fiber, e.g. the MPE, may further be evaluated, e.g. quantified, by analyzing the fermentation time of the tested fiber, and/or the relationship between the faecal bacterial population growth and the substrate, e.g. fiber, concentration.

According to the invention the MPE may include, e.g. may be defined by, the Prebiotic Index, as hereinabove described.

In another aspect of the invention, the MPE may include, e.g. may be defined by, the fermentation pattern of the tested fiber, e.g. the measure of the fermentation end products, e.g. short chain fatty acids (SCFA), e.g. when the feacal bacterial population is at the end of the exponential growth phase. The MPE may further include, e.g. may be defined by, the relationship between the production of fermentation end products, e.g. SCFA, and the concentration of substrate, e.g. fiber.

According to the invention, the measure of the fermentation end products may include, e.g. may consist in, calculating the mass, e.g. production, of acetate, propionate, butyrate and lactate produced by the faecal bacteria, e.g. at the end of the exponential growth phase. The following equation may be used:

$$T_{SCFA} = A + B + P + L$$

wherein $T_{SCFA}$ = production of SCFA; A = amount of acetate produced; B = amount of butyrate produced; P = amount of propionate produced and L = amount of lactate produced.

According to the invention, the measure of the fermentation end products may include, e.g. may consist in, calculating the ratio of lactic acid production, e.g. at the end of the exponential growth phase of the feacal bacteria population, over the total SCFA production, e.g. over the production of acetate, propionate, butyrate and lactate, e.g. as hereinabove described. This ratio may be calculated as follows:

$$\text{Ratio} = dL / dT_{SCFA}$$

wherein d is the difference between the initial mass and mass at sampling time point, dL= amount of lactic acid at time t minus the amount of lactic acid at time zero, and $dT_{SCFA}$ = amount of SCFA, e.g. acetate, propionate, butyrate and lactate, at time t minus the amount of SCFA at time zero.

In a further aspect of the invention, the MPE may include, e.g. may be defined by, the measure of the substrate, e.g. fiber, assimilation, e.g. by the measure of the rate of substrate assimilation.

In one aspect of the invention, the measure of the substrate assimilation may be carried out by measuring the substrate concentration over time. The following equation may be used:

$$S_t = S_0 - A_r t$$

wherein $S_t$ = substrate concentration after the time interval, $t$, e.g. in hours; So = initial substrate concentration and $A_r$ = rate of substrate assimilation, e.g. per hour, e.g. during the exponential phase of bacterial population growth.

In yet a further aspect of the invention, the MPE may include, e.g. may be defined by, the determination of the fermentation time, e.g. the time necessary for the fiber to be fermented, e.g. completely fermented. The MPE may include, e.g. may be defined by, the relationship between the faecal bacterial population growth and the substrate, e.g. fiber, concentration.

In step (d) of the method according to the invention, the equations measuring the substrate assimilation, the analysis of the faecal bacterial population, e.g. the PI, and the measure of the fermentation end product, e.g. the ratio as hereinabove described, are combined in one single equation, which result in a single number, the MPE value.

According to the method of the invention, the MPE is described by the following equation:

$$\frac{1}{2}\sqrt{x^2 y^2 + x^2 z^2 + y^2 z^2} = MPE$$

wherein

x = changes in the bacterial population induced by the tested fiber, e.g. as measured by PI;

y= quantification of the fermentation end products production, e.g. SCFA production, e.g. acetate, propionate, butyrate and lactate production, or relationship between fermentation end products production, e.g. SCFA production, and substrate concentration, e.g. fiber concentration;

z= rate of substrate assimilation, e.g. fiber breakdown, e.g. relationship between substrate, e.g. fiber, concentration and bacterial population growth. Z may be defined with the above mentioned equation.

The parameter y may also be the ratio of lactate production over total SCFA production, e.g. over production of acetate, butyrate, propionate and lactate, as hereinabove described.

According to the method of the invention, it is possible to attribute to the calculated MPE value a positive or negative sign, e.g. the sign of the PI as calculated for the same fiber, of fibers blend, in the same conditions, i.e. to attribute a positive sign to the MPE of a fiber for which the PI is positive, e.g. for the MPE calculated for FOS, and reciprocally, a negative sign to the MPE of a fiber for which the PI is negative, e.g. for the MPE calculated for sucrose.

As used herein, "faecal bacteria" refers to indigenous or commensal bacteria of the colon, gut and long intestine, e.g. predominant bacterial groups present in the faeces, including beneficial and potentially beneficial bacteria, such as bifidobacteria, lactobacilli, eubacteria, and non beneficial, e.g. detrimental, putrefactive or pathogenic bacteria, such as bacteroides, clostridia, coliforms, Sulfate Reducing bacteria (SRB). Faecal bacteria may be single bacteria species or may be mixtures of different species. Faecal bacteria may derive from natural sources, such as faecal samples from human, e.g. healthy human and/or human who did not take any antibiotics for at least 6 months. Faecal bacteria may be or not purified.

For the purpose of the invention, the term " fiber" refers to fibers, e.g. dietary fibers, e.g. soluble or insoluble fibers, e.g. hydrolyzed fibers. In particular the fibers according to the invention are able to undergo fermentation in the colon to produce short chain fatty acids (SCFA), such as e.g. succinic, lactic, formic, acetic, propionic, isobutyric, butyric, isovaleric or valeric acids or hydrogen and carbon dioxide gases.

Examples of fibers according to the invention are fructooligosaccharides (also called oligofructose) (FOS), glucooligosaccharides, galactooligosaccharides (GOS), guar gum, hydrolyzed guar gum, isomaltooligosaccharides (IMO), soyoligosaccharides (SOS), and mixtures thereof.

FOS are members of the inulin subclass of fructans. FOS occur in nature in many kind of plants, including onions, garlic, shallots, wheat, rye, bananas, aspergus, tomatoes, artichokes, dahlia and chicory root. FOS can be produced enzymatically, through chemical techniques or by extraction from natural substances. Short chain FOS are composed of one to three fructose molecules linked to one molecule of sucrose: their polymerization degree (DP) is not higher than 6, and they can be synthesized from sucrose through the use of transfructosylating enzymes. Treatment of sucrose with these transfructosylating enzymes results in a mixture of FOS containing 2, 3 or 4 fructose units, such as 1-kestose, nystose and fructosyl-nystose. *In vivo* human studies have been shown that dietary addition of FOS leads to an increase in faecal bifidobacteria and is a very effective prebiotic.

As used herein the term "FOS" encompasses FOS and short chain FOS. FOS may comprise between 2 and 20 saccharide units, for example between 2 to 15 saccharide units, further example between 2 to 7 saccharide units or between 2 to 6 saccharide units. For example FOS may contain about 95% by weight disaccharides to heptasaccharides, based on the total weight of FOS.

Oligofructose is commercially available, for example as Actilite, RAFTILOSE&commat; from ORAFTI, (Tienen, Belgium), in various grades such as, for example, RAFTILOSE&commat; P95 which contains about 95 % by weight oligofructose, composed of chains with a degree of polymerisation ranging from 2 to about 7, typically with a (DP) of 3.5 to 4.5, and containing about 5 % by weight in total of glucose, fructose and sucrose.

According to the invention GOS may comprise di, tri, tetra, penta and hexasaccharides, mainly consist of galactose as a sugar component, and are formed by the action of beta-galactosidase on lactose. GOS may comprise between 2 and 15 saccharide units, for example between 2 to 10 saccharide units, further example between 2 to 7 saccharide units or between 2 to 6 saccharide units. For example GOS may contain about 0 to about 45% of weight disaccharides, further example about 10 to about 40% of weight disaccharides, about 20 to about 35% of weight disaccharides, or about 33% of weight disaccharides, based of the total weight of GOS. For example GOS may contain about 0 to about 50% of weight trisaccharides, further example about 10 to about 45% of weight trisaccharides, about 20 to about 40% of weight trisaccharides, or about 39% of weight trisaccharides, based of the total weight of GOS. For example GOS may contain about 0 to about 50% of weight tetrasaccharides, further example about 5 to about 45% of weight tetrasaccharides, about 10 to about 40% of weight tetrasaccharides, or about 18% of weight tetrasaccharides, based of the total weight of GOS. For example, GOS may contain about 0 to about 30% of weight pentasaccharides, further example about 1 to about 25% of weight pentasaccharides, about 2 to about 10% of weight pentasaccharides, or about 7% of weight pentasaccharides, based of the total weight of GOS.

GOS is commercially available, for example under the trade name Vivinal GOS or Elix'or GOS.

As used herein the term "GOS" encompasses GOS as hereinabove defined and trans Galactooligosaccharides, also called tGOS.

Hydrolysed fiber may be derived from numerous known fibers. Preferred hydrolysed fibers include hydrolysed guar gum, e.g. partially hydrolyzed guar gum. The term hydrolysed fibers as used herein refers to fibers hydrolysed in conventional manner, e.g. chemically or enzymatically to fibers having a reduced molecular weight, which hydrolysed products may be tube compatible when administered at the desired daily amount.

An example of hydrolysed guar gum is Benefiber®, e.g. as described in U.S. Patent No.5,260,279, which is hereby incorporated by reference. Prior to hydrolysis, the molecular weight of guar gum is approximately 200,000; after hydrolysis it is 20,000-30,000. The molecular weight range of the hydrolysed guar gum may vary, preferably may be between 24 and 30 kDa.

IMO and SOS are commercially available, e.g. from Showa Sangayo, Japan, and Calpis, Japan, respectively.

For the method of the present invention anaerobic culture fermentations, such as batch culture fermentations, may be used to assess and measure e.g. bacterial growth or changes in bacterial population, e.g. through the calculation of the PI; fermentation times; rate of substrate assimilation, e.g. fiber breakdown, e.g. in relation to bacterial growth; production of fermentation end products, e.g. SCFA; relationship between fermentation end products production and substrate concentration; ratio of lactate over total SCFA or over acetate, butyrate, propionate and lactate or MPE as hereinabove described.

Batch culture fermentations may also be used for the method of the invention to determine the minimum and maximum concentration of substrate, e.g. fiber, the prebiotic capability, e.g. the PI, or the MPE, of combination of test substrates, e.g. combinations of test fibers, such as FOS: GOS, e.g. FOS: GOS (50:50), FOS: Benefiber®, e.g. FOS: Benefiber® (90:10), and GOS: Benefiber®, e.g. GOS: Benefiber® (90:10). Sucrose may be used as a control substrate as it is not selective and will therefore be fermented by all bacteria. As a negative control culture medium without any substrate may be used.

Preferably, the batch culture fermentation is carried out in the presence of about 0.5 to about 3% by weight, e.g. about 1 to about 2% by weight, e.g. about 1% by weight, of the tested fiber, or blend of tested fibers, e.g. 1 % by weight FOS, based on the total weight of the batch culture.

As used herein, "batch fermentation culture" refers to anaerobic culture inoculated with faecal slurry and containing the tested fiber, or blend of tested fibers, or sucrose. According to the method of the invention, the nutrient medium of the batch fermentation culture can be gassed with oxygen-free nitrogen, e.g. overnight, before adding any feacal bacteria, and the culture can be maintained in a continuous oxygen-free nitrogen environment, e.g. by being continuously sparged with $O_2$-free $N_2$, e.g. at a flow rate of 15ml/min.

According to the method of the invention, the faecal sample may be taken from human, e.g. healthy human, e.g. human who had no history of any gastrointestinal diseases, and/or human who did not take any antibiotics for at least 6 months. The faecal sample may be diluted, e.g. at about 1/10, in anaerobic buffer, e.g. anaerobic phosphate buffer, e.g. containing 0.1 M phosphate and having a pH 7.4. The faecal sample may be homogenized.

The incubation of the faecal bacterial culture may be carried out in a volume comprised between about 100ml and 500ml, e.g. between about 100 ml and 400 ml, e.g. between 150 and 300ml, e.g. about 150ml. Preferably the incubation volume may be about 150 ml, or about 250ml, or about 300ml, e.g. 270ml.

The culture temperature can be comprised between about 35 and 42°C, preferably can be about 37°C.

The culture pH can be comprised between 6.5 and 7, preferably about 6.8.

The incubation of the faecal bacterial culture may be carried out until the substrate, e.g. the fiber, has been fermented, e.g. completely fermented. The incubation period may be of about 24 hours.

According to the invention, gut model experiments, e.g. three-stage thermostat experiments, may also be used e.g. to assess the persistence of test substrates, e.g. fibers, e.g. combination of fibers, through the colon; to assess the effect of each substrate, e.g. fiber, on bacterial growth and/or SCFA production. The gut model has been validated against gut contents from sudden death victims and gives a very close analogy to bacterial composition and activities in different areas of the large intestine. With the gut model, a period of fermentation may last for at least one day and up to about 15 days, e.g. between 1 day and 11 days, e.g. between 10 and 11 days.

For the method of the present invention bacterial population growth and changes may be determined by fluorescence *in situ* hybridisation (FISH), a culture independent molecular technique employing 16S rRNA oligonucleotide probes labelled with fluorescent dyes (table1). The FISH method allows the visualization and localization of whole bacterial cells *in situ* in environmental samples. It will be appreciated that such a method is readily known to one skilled in the art.

For the method of the present invention, the production of SCFA may be determined by a technique readily known to one skilled in the art, such as HPLC.

For the method of the present invention, the measure of the total carbohydrate content, e.g. the measure of the substrate concentration, may be determined by phenol-sulphuric acid assay. The assay can be calibrated with e.g. D-glucose standards, e.g. ranging from 0 to 0.15mg/ml. It will be appreciated that such a method is readily known to one skilled in the art.

A prebiotic may be included in a nutritional or pharmaceutical composition. Nutritional compositions refer to nutritional formulations, typically nutraceuticals, dietary supplements, functional food, beverage products, or food additives. Such nutritional compositions may be nutritionally complete, i.e. may include vitamins, minerals, trace elements as well as nitrogen, carbohydrate and fatty acid sources so that they may be used as the sole source of nutrition supplying essentially all the required daily amounts of vitamins, minerals, carbohydrates, fatty acids, proteins and the like. The nutritional compositions may also be in the form of low calorie formulations, e.g. low calorie meal replacements.

The compositions containing a prebiotic may be suited for oral or tube feeding.

Suitable product formats for the nutritional compositions include solutions, ready-for-consumption compositions, e.g. ready-to-drink compositions, instant drinks, liquid comestibles, like soft drinks, juices, sports drinks, milk drinks, milk-shakes, yogurt drinks or soups. Such compositions may also be designed in the form of a concentrate, a powder, or granules, e.g. effervescent granules, to be diluted in water or other liquid, such as milk or fruit juice.

Pharmaceutical compositions may be provided in the form of soft gels, sachets, powder, syrups, liquid suspensions, emulsions, solution, hard gelatin capsules or soft, sealed capsules consisting of gelatin and a plasticizer, such as glycerol or sorbitol.

The amount of fiber, e.g. prebiotics, contained in the compositions described above may be determined in the light of various relevant factors including the purpose of administration, the age, sex and body weight of individual subject and the severity of the subject's symptoms.

The compositions described above may also comprise any bioactive compounds or extracts which are known to have health benefits, especially compounds which have a beneficial influence on the gastro-intestinal tract, such as probiotics, glutamine/glutamate or precursors thereof, or that inhibit bacterial adhesion to epithelial wall of the gastrointestinal tract, including mannans, galacturonic acid oligomers, preferably of natural origin.

The invention may be further illustrated by the following Examples:

Examples

Unless otherwise stated, all chemicals and reagents are obtained from Sigma - Aldrich Co. Ltd. (Poole-UK) or BDH Chemicals Ltd. (Poole, UK).

Where required, culture pH is adjusted with either 1 M HCl or 1M NaOH. Sterilisation is achieved by autoclaving at 121°C for 15 min.

The fructooligosaccharide (FOS) is Actilight 950P®, Eridania Beighin Meiji, Neuilli sur Seine, France (containing 95% oligosaccharides),

The trans-Galactooligosaccharide (tGOS) is Elix'or®' Borculo Domo, Netherlands (containing 58% tGOS, 19% glucose, 19% lactose, 0.8% galactose, 3% moisture)

The partially hydrolysed guar gum (PHGG) is Benefibre® from Novartis Nutrition Corporation or Sunfiber (HM 1) from Taiyo Kaguku Co., Yokkaichi, Japan.

The isomaltooligosaccharide (IMO) is obtained from Showa Sangayo, Japan.

The soyoligosaccharide (SOS) is obtained from Calpis, Japan.

(1) Preparation and collection of faecal samples

Faecal samples are obtained from healthy human volunteers. Volunteers are required not to have been prescribed antibiotics for at least 6 months prior to the study and have no history of gastrointestinal diseases. The samples are collected on site and used immediately following collection. A 1/10 dilution in anaerobic phosphate buffer (0.1M, pH 7.4) is prepared and the samples homogenised in a stomacher for 2 minutes.

(2) Example 1: Batch Fermentations

Sterile, stirred, batch culture fermentation vessels (300ml volume) are filled with 135ml basal nutrient medium (peptone water 2g/l, yeast extract 2g/l, NaCl 0.1g/l, $K_2HPO_4$ 0.04g/l, $KH_2PO_4$ 0.04g/l, $MgSO_4.7H_2O$ 0.01g/l, $CaCl_2.6H_2O$ 0.01, $NaHCO_3$ 2g/l, Tween 80 2ml, Hemin 0.02g/l, Vitamin $K_1$ 10µl, Cysteine.HCl 0.5g/l, Bile salts (sodium glycocholate and sodium taurocholate) 0.5g/l, pH7.0) and gassed overnight with oxygen free nitrogen. Prior to addition of the faecal slurry,0.25%, 0.5% or 1% (w/v) testing substrates are added to different vessels, culture temperature is set at 37°C by means of a circulating water bath and medium pH is maintained at 6.8 using an Electrolab pH controller. The vessels are inoculated with 15ml of fresh faecal slurry (1/10w/v) and continuously sparged with $O_2$-free $N_2$ at a flow rate of 15ml/min. Samples (3ml) from each vessel are obtained for fluorescence *in situ* hybridisation (FISH), analysis of SCFA by high performance liquid chromatography (HPLC) and total carbohydrate measurement by assay. Batch cultures are run over a period of 24 hours and samples are obtained every 2 hours up to 12 hours and then at 15 and 24 hours.

2.1. Bacterial enumeration: Fluorescence *in situ* hybridisation (FISH)

Differences in bacterial populations are assessed through use of FISH with oligonucleotide probes designed to target diagnostic regions of 16S rRNA. These are commercially synthesised and labelled with the fluorescent dye Cy3 (provided by Eurogentec UK Ltd). The molecular probes utilised are presented in Table 1.

Table 1:

| Probe | Sequence | Target genus | T° |
|---|---|---|---|
| Bac 303 | 5'-CCAATGTGGGGGACCTT-3' | *Bacteroides* spp. | 45°C |
| Bif 164 | 5'-CATCCGGCATTACCACCC-3' | *Bifidobacterium* spp. | 50°C |
| Erec 482 | 5'-CGGUACCUGACUAAGAAGC-3' | *Clostridium coccoides- Eubacterium rectale* group | 50°C |
| Chis 150 | 5'-AAAGGAAGAUUAAUACCGCAUA-3' | *Clostridium histolyticum* group | 50°C |
| Ec 1531 | 5'-CACCGTAGTGCCTCGTCATCA-3' | *E.coli* | 37°C |
| Lab 158 | 5'-GGTATTAGCA(T/C)CTGTTTCCA-3' | *Lactobacillus*/*Enterococcus* spp. | 45°C |

(continued)

| Probe | Sequence | Target genus | T° |
|---|---|---|---|
| Srb 687 | 5'- TACGGATTTCACTCCT-3' | *Desulfovibrio spp.* | 48°C |

For total bacterial counts the nucleic acid stain 4,6-diamidino-2-phenylindole (DAPI) is used. Samples obtained from fermentation vessels are diluted in 4% (w/v) paraformaldehyde and fixed overnight at 4°C. The cells are then centrifuged at 1500 x g for 5 minutes, washed twice with phosphate-buffered saline (PBS; 0.1M, pH 7.0), resuspended in a mixture of PBS / 99% ethanol (1:1 w/v) and stored at -20°C for at least 1 hour. For Lab 158 probe samples are further resuspended in the enzyme mixture containing agents that increase cell permeability and incubated for 1 hour at 37°C. The cells are then washed in PBS, resuspended in 100% methanol and stored at -20°C for at least 1 hour. The cell suspension is then added to the hybridisation mixture and left overnight to hybridise at the appropriate temperature for each probe. Hybridized mixture is vacuum filtered using a $0.2\mu$m Isopore membrane filter (Millipore Corporation, Herts, UK). The filter is removed, placed onto a glass slide with SlowFade (Molecular Probes, Eugan, OR, USA) and examined under a fluorescent microscope (Nicon Eclipse, E400). The DAPI stained cells are examined under UV light and hybridised cells viewed using a DM510 filter. Faecal sample are incubated with 1% (w/v) of each substrate. Samples are taken after 24 hours. For each slide at least 15 different fields of view are counted. Microbial counts are presented as $log_{10}$cells/ml.

2.1.1. PI

PI is calculated with the following equation: PI = $\Delta$B + $\Delta$L + $\Delta$E - $\Delta$Ba - $\Delta$Cl - $\Delta$Co - $\Delta$SRB, wherein $\Delta$ = amount of bacteria in presence of the tested fiber minus the amount of bacteria in absence of the tested fiber; B = *Bifidobacterium* spp; L = lactobacilli; E = *Eubacterium* rectale; Ba = *Bacteroides* spp; Cl = *Clostridium* coccoides and *Clostridium* histolyticum; Co = *Escherichia* coli and SRB = *Desulfovibrio* spp.

Results (table 2)

| Bacteria | Sucrose | FOS | $\Delta$FOS | tGOS | $\Delta$tGOS | PHGG | $\Delta$PHGG |
|---|---|---|---|---|---|---|---|
| Bifidobacteria | 8.0 | 8.9 | 0.9 | 8.7 | 0.7 | 8.4 | 0.4 |
| Bacteroides | 8.1 | 8.2 | 0.1 | 7.8 | -0.3 | 9.0 | 0.9 |
| Lactobacilli | 6.7 | 7.0 | 0.3 | 7.0 | 0.3 | 6.8 | 0.1 |
| Clostridia | 7.6 | 7.3 | - 0.3 | 6.9 | - 0.7 | 6.8 | -0.8 |
| E.Coli | 7.7 | 7.8 | 0.1 | 6.9 | -0.8 | 7.5 | -0.2 |
| Eubacteria | 7.7 | 8.0 | 0.3 | 7.7 | 0 | 8.2 | 0.5 |
| Desulfovibrio | 6.7 | 6.9 | 0.2 | 7.2 | 0.5 | 6.9 | 0.2 |
| PI | | | 1.4 | | 2.3 | | 0.9 |

tGOS shows the highest growth rate of *Bifidobacterium* spp. at the expense of *Bacteroides* spp. and *Eubacterium rectale/Clostridium coccoides* group, as well as the smallest increase in *E. coli*. FOS also results in a high growth rate of bifidobacteria, however, high growth rates of *Clostridium histolyticum* group and *E. coli* are also observed. PHGG (Benefiber®) results in a high growth rate of *Bacteroides* spp. and *E.coli* with a positive effect on bifidobacteria when compared to sucrose.

2.1.2. PI

PI is calculated with the following equation:

$$PI = \mu_{max} B + \mu_{max} L + \mu_{max} E - \mu_{max} Ba - \mu_{max} Cl - \mu_{max} Co - \mu_{max}SRB;$$

wherein $\mu_{max}$ = maximum growth rates of bacteria, B = *Bifidobacterium* spp; L = lactobacilli; E = *Eubacterium* rectale; Ba = *Bacteroides* spp; Cl = *Clostridium* coccoides and *Clostridium* histolyticum; Co = *Escherichia* coli and SRB = *Desulfovibrio* spp.

1% (w/v) of the following substrates is used: sucrose, guar gum, FOS, tGOS, Benefibre®, Sunfiber, combination FOS and Benefiber® (90/10), combination tGOS and Benefiber® (90/10) and combination FOS and tGOS (50/50).

Results (Table 3)

| | μmax (h-1) | | | | | | | |
|---|---|---|---|---|---|---|---|---|
| | B | L | E | Ba | Cl | Co | SRB | PI |
| Sucrose | 0.13 | 0.03 | -0.21 | 0.11 | 0.22 | 0.27 | 0.21 | -0.9 |
| Guar Gum | 0.21 | 0.07 | 0.10 | 0.25 | 0.47 | 0.35 | 0.13 | -0.8 |
| PHGG-Sunfiber | 0.10 | 0.15 | 0.11 | 0.08 | 0.06 | 0.28 | 0.21 | -0.3 |
| PHGG_Benefiber® | 0.10 | 0.03 | 0.05 | 0.24 | -0.01 | 0.04 | 0.03 | -0.1 |
| IMO | 0.33 | 0.25 | 0.07 | 0.06 | 0.25 | 0.35 | -0.11 | 0.1 |
| SOS | 0.34 | 0.24 | 0.04 | 0.07 | 0.10 | 0.31 | 0.02 | 0.1 |
| FOS | 0.35 | 0.06 | -0.02 | -0.02 | 0.03 | 0.13 | 0.10 | 0.2 |
| FOS/Benefiber® | 0.30 | 0.13 | 0.14 | 0.04 | -0.01 | 0.18 | 0.03 | 0.3 |
| tGOS/Benefiber® | 0.28 | 0.13 | 0.11 | 0.05 | -0.02 | 0.08 | 0.08 | 0.3 |
| tGOS | 0.60 | 0.02 | -0.09 | -0.33 | 0.03 | 0.04 | 0.04 | 0.8 |
| FOS/tGOS | 0.47 | 0.09 | 0.13 | -0.05 | -0.23 | -0.27 | -0.05 | 1.3 |

Different PI values are obtained for the different substrates. Sucrose, guar gum, Sunfiber and Benefiber® all have a negative value whilst the assessment of rest of the substrates and combinations is positive. Positive or negative PI value is determined by the proportion of desired bacterial groups (in this case bifidobacteria, lactobacilli and eubacteria) versus less desirable bacterial groups. The extent of PI value is determined by the selectivity of each substrate. For example, both guar gum and Benefiber® have a negative value, however guar gum supports growth of most bacterial groups assessed whereas Benefiber® supports growth of mainly bacteroides and to some extent bifidobacteria. Guar gum is less selective and thus results in much lower PI value than Benefiber®. Similarly tGOS and SOS both have a positive PI value; tGOS is almost exclusively selective towards bifidobacterial growth and results in a very high PI. SOS supports growth of both bifidobacteria and lactobacilli but also of *E. coli* and clostridia; it results in much lower PI value than tGOS.

2.2 Analysis of SCFA production

The production of succinic, lactic, formic, acetic, propionic, isobutyric, butyric, isovaleric and valeric acids in the fermentations is quantified by HPLC. Samples are centrifuged at 1500 x g for 15 minutes and the resultant supernatant used for injection. A Model 1050 UV HPLC (Hewlett Packard), with an integrated oven compartment (50°C) and data system is used in combination with a differential refractometer (Knauer). Sample injection is performed using an autosampler and is of 20μl volume. The column is a pre-packed Aminex HPX-87-H strong cation-exchange resin column (150 x 7.8 mm I.D.), fitted with an ion exclusion micro-guard refill cartridge (Bio-Rad Labs., USA). The eluent used is 0.005 M sulphuric acid. Faecal samples are incubated with 135ml basal nutrient medium containing 1% (w/v) substrate in batch culture systems at 37° C. Samples are taken every 2 hours up to 10 hours and then at 15 and 24 hours. Data are not shown.

The ratio of lactate over the total SCFA production is then calculated, at the end of exponential phases (10 hours in the case of FOS, FOS/ PHGG-Benefiber®, guar gum and PHGG-Sunfiber, 8 hours in the case of sucrose, tGOS, tGOG/FOS, tGOS/ PHGG-Benefiber®, IMO and SOS, and 15 hours in the case of PHGG-Benefiber®).

Results: Sucrose, PHGG-Benefiber® , guar gum, PHGG -Sunfiber and IMO fermentations result in low lactate production and the calculated ratio for these substrates is very low. FOS,tGOS on their own and in combination as well as SOS, result in higher ratio showing that these substrates better support the growth of lactic acid producing bacteria.

2.3. Measurement of total sugars by total carbohydrate assay.

A phenol-sulphuric acid assay is used for the determination of total carbohydrate content as expressed in glucose equivalents. The assay is calibrated with D-glucose standards ranging from 0 to 0.15 mgml-1. Measurements

of the absorbance at 450nm are taken using the spectrophotometer and plotted against the standards. The total carbohydrate content is thus calculated.

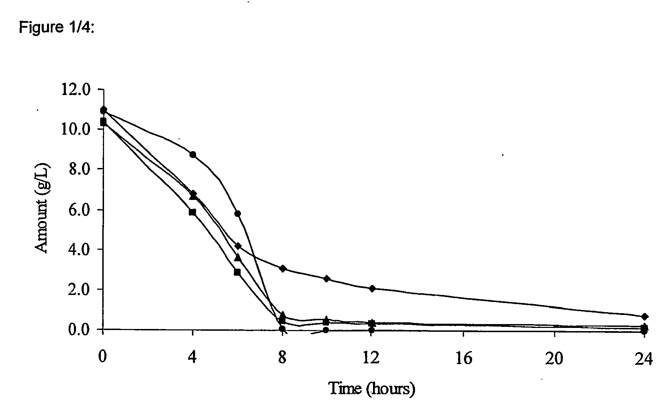

Faecal samples are incubated with 0% (w/v), 0.25% (w/v), 0.5% (w/v) and 1% (w/v) of substrate and 135ml basal nutrient medium in batch culture systems at 37° C. Samples are taken every 2 hours up to 10 hours and then at 15 and 24 hours. Figure 1 shows an example of such measurements for 1% (w/v) sucrose, guar gum, FOS and tGOS.

The rate of assimilation is calculated with the following equation: $S_t = S_0 - A_r t$

wherein $S_t$ = substrate concentration after the time interval $t$ in hours (calculated every two hours, between 2 and 8 hours for sucrose, Benefiber® and FOS and between 4 and 8 hours for tGOS), So = 1 % by weight, based on the total weight of the culture, and $A_r$ = rate of substrate assimilation per hour during the exponential phase of bacterial growth.

Results (table 4):

|  | $A_r$ | Ratio | MPE |
|---|---|---|---|
| Sucrose | 1.4 | 0.1 | **-0.6** |
| Guar Gum | 1.0 | 0.2 | **-0.4** |
| Sunfiber | 0.8 | 0.2 | **-0.2** |
| Benefiber® | 0.7 | 0.1 | **-0.1** |
| IMO | 1.2 | 0.2 | **0.1** |
| FOS/ Benefiber® | 0.7 | 0.3 | **0.2** |
| tGOS/ Benefiber® | 0.7 | 0.3 | **0.2** |
| SOS | 1.2 | 0.4 | **0.3** |
| FOS | 1.4 | 0.5 | **0.4** |
| tGOS | 2.2 | 0.4 | **1.0** |
| FOS/tGOS | 1.9 | 0.5 | **1.4** |

The measure of the concentration of total carbohydrates present in each fermentation vessel shows variation in fermentation times between different substrates. tGOS followed by tGOS/FOS results in a fast, whereas PHGG - Benefiber® on its own and combined with either FOS or tGOS, followed by PHGG-Sunfiber results in slow fermentation. The $A_r$ values are similar for sucrose and FOS, i.e. $A_r$= 1.40, indicating that these two substrates have similar fermentation times.

2.4. MPE values

The three values (PI, $A_r$ and ratio) obtained are used to calculate the MPE value using the following equation:

$$\frac{1}{2}\sqrt{x^2 y^2 + x^2 z^2 + y^2 z^2} = MPE$$

wherein x = PI (as calculated under paragraph 2.1.2); y = ratio of lactate over total SCFA (as calculated under paragraph 2.2) and z = $A_r$ (as calculated under paragraph 2.3).

The ratio of lactate is calculated at the time point where maximum production of lactic acid occurs, i.e. about 8 hours for sucrose and GOS and about 10 hours for FOS.

Positive or negative value of the MPE is determined by the PI value.

Results (table 4 above)

Comparison of similar and/or different substrates can be achieved. For example, in the case of sucrose and guar gum PI values are similar and lower MPE is obtained for sucrose, because its $A_r$ value is higher. Similarly, IMO and SOS have similar PI and $A_r$ values, and higher MPE is obtained for SOS because SOS better supports the growth of lactic acid producing bacteria than IMO and its Ratio is higher.

The MPE values obtained here indicate that tGOS/FOS combination followed by tGOS and FOS produce the best *in*

*vitro* prebiotic effect of the substrates tested.

(3) Example 2: *In vitro* gut model

The conditions in the colon are replicated in a three stage continuous fermenter (Macfarlane *et al*., 1998) inoculated with 10% (w/v) faecal homogenate from healthy human volunteers in a growth medium without and with 1 % (w/v) testing substrate. The model consists of three vessels, V1, V2 and V3, with respective operating volumes of 270, 300 and 300 ml. Temperature is set at 37°C and together with pH is controlled automatically. Culture pH in the three vessels is maintained at 5.5, 6.2 and 6.8, respectively. Each fermenter is magnetically stirred and kept under anaerobic conditions by continuously sparging with $O_2$-free $N_2$ (15ml/min). The growth medium contains the following ingredients: starch 8g/l, mucin 4g/l, casein 3g/l, peptone water 5g/l, tryptone water 5g/l, bile N°3 0.4g/l, yeast, 4.5 g/l, $FeSO_4$ 0.005g/l, NaCl 4.5g/l, KCl 4.5g/l, $KH_2PO_4$ 0.5g/l, $MgSO_4.7H_2O$ 1.25g/l, $CaCl_2.6H_2O$ 0.15g/l, $NaHCO_3$ 1.5g/l, Tween 80 1ml, Hemin 0.05g/l, Cysteine.HCl 0.8g/l. The medium is fed to V1 by a peristaltic pump and V1 sequentially supplies V2 and V3 through a series of tubes. The system is operated at a retention time of about 36 hours. The gut model is left overnight to equilibrate before the medium pump is switched on and is run for 10.5 days before medium containing testing substrate is introduced and it is then left for further 10.5 days. Samples are taken at the beginning and the end of each cycle. The sample volume removed is 5 ml and this amount is used for SCFA analyses, FISH and total carbohydrate measurement.

3.1. Fluorescence *in situ* hybridisation (FISH)

Faecal sample are incubated with 1% (w/v) of each substrate. Samples are taken after 21 days. For each slide at least 15 different fields of view are counted. Microbial counts are presented as $log_{10}$cells/ml.

PI is calculated with the following equation:

$$PI = \Delta B + \Delta L + \Delta E - \Delta Ba - \Delta Cl - \Delta Co - \Delta SRB$$

Wherein $\Delta$ = amount of bacteria in presence of the tested fiber minus the amount of bacteria in absence of the tested fiber; B = bifidobacteria; L = lactobacilli; E = Eubacteria; Ba = bacteroides; Cl = clostridia; Co = E coli and SRB = Desulfovibrio.

Total PI is calculated by summing PI for each vessel. The base line for these values is gut model medium.

|          | Gut model medium | | | FOS | | | tGOS | | | PHGG | | |
|----------|------|------|------|------|------|------|------|------|------|------|------|------|
| Bacteria | V1   | V2   | V3   | V1   | V2   | V3   | V1   | V2   | V3   | V1   | V2   | V3   |
| B        | 8.1  | 8.0  | 8.0  | 8.4  | 8.3  | 8.1  | 8.3  | 8.3  | 8.3  | 8.0  | 8.0  | 8.2  |
| Ba       | 8.0  | 8.2  | 8.1  | 7.7  | 7.7  | 7.8  | 7.5  | 7.5  | 7.5  | 8.0  | 8.7  | 8.7  |
| L        | 7.1  | 7.0  | 6.9  | 7.1  | 7.1  | 7.0  | 7.1  | 7.1  | 6.9  | 7.1  | 7.1  | 7.0  |
| Cl       | 6.8  | 6.8  | 6.9  | 6.8  | 6.8  | 6.8  | 7.1  | 7.1  | 7.0  | 7.2  | 6.9  | 6.6  |
| Co       | 6.9  | 6.9  | 7.1  | 7.2  | 7.0  | 7.1  | 7.2  | 7.1  | 7.2  | 7.0  | 7.2  | 7.2  |
| E        | 7.7  | 7.9  | 8.0  | 7.5  | 7.6  | 7.4  | 7.5  | 7.5  | 7.5  | 7.8  | 7.4  | 8.3  |
| SRB      | 7.2  | 7.2  | 7.3  | 7.0  | 7.2  | 7.2  | 6.7  | 6.9  | 6.9  | 7.6  | 7.7  | 7.8  |

| PI       | FOS  | tGOS | PHGG |
|----------|------|------|------|
| V1       | 0.4  | 1.6  | -0.9 |
| V2       | 0.5  | 1.0  | -1.6 |
| V3       | 1.0  | 0    | -0.3 |
| Total PI | 1.9  | 2.6  | -2.8 |

Results: From both batch cultures and gut models, tGOS seems to have the best effect. tGOS and FOS have a similar PI in both batch and gut models.

3.2. Analysis of SCFA

The same protocol is used as for batch fermentation. The SCFA profiles measured by HPLC for gut models containing 1% (w/v) partially hydrolyzed guar gum (PHGG) (Benefiber®), FOS (Actilight) and tGOS (Elix'or) as substrates are presented in Figures 2, 3 and 4, respectively. The gut model is ran for the first 10.5 days with gut model medium after which time medium is supplemented with subtrate and gut model is ran for the further 10.5 days. Three vessels of the model correspond to pH 5.5, pH 6.2 and pH 6.8. ■ acetic, ♦ lactic, A propionic and • butyric acid.

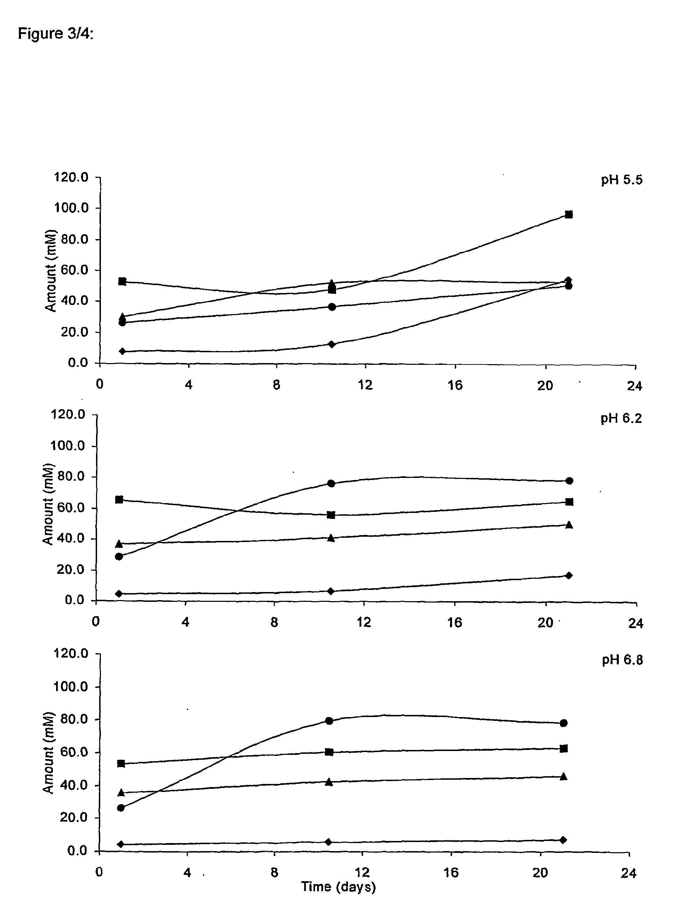

Results: In general, lactic acid production is low throughout all vessels and with all substrates assessed. The only increase is observed with the addition of FOS in the first vessel (Figure 3a). Similarly, acetic acid production is increased only in the first vessel in the gut model containing FOS (Figure 3a) and decreased in the first vessel containing PHGG (Figure 2a), whilst the rest shows no change. The addition of FOS and tGOS into the medium results in no observed effect upon production of propionic acid in any of the vessels, whilst the addition of PHGG results in increased production of propionic acid in all three vessels. Butyric acid production is increased with the addition of tGOS and PHGG in all three vessels. The addition of FOS has no effect on the production of butyric acid in any vessel. Generally, the SCFA profiles demonstrates that both tGOS and PHGG in particular, have an effect in all three vessels whilst FOS has the most pronounced effect on the first vessel and very little effect upon the second and third vessels.

**Claims**

1. A method for evaluating or quantifying the prebiotic capability of a fiber or for identifying a prebiotic substance, which comprises

   (a) a step of evaluating or quantifying the stimulation by the tested fiber or substance on the growth and/or modification of a faecal bacterial population,
   (b) a step of quantifying at least one product resulting from the fermentation of the tested fiber or substance,
   (c) a step of quantifying the rate of assimilation of the tested fiber or substance,
   (d) a step of calculating the Measure of the Prebiotic Effect (MPE) using the following equation:

$$\frac{1}{2}\sqrt{x^2 y^2 + x^2 z^2 + y^2 z^2} = MPE$$

   wherein x is the value as determined in step (a), y is the value as determined in step (b) and z is the value as determined in step (c), and
   (e) a step of evaluating or quantifying the prebiotic capability of the tested fiber or substance as a function of the MPE.

2. The method according to claim 1 which comprises

   (a) incubating the faecal bacterial culture in the presence of the tested fiber or substance, and
   (b) determining the total amount and/or maximum growth rate of the beneficial faecal bacteria population, and
   (c) evaluating or quantifying the effect on the growth and/or modification of the faecal bacterial population as a function of the value as determined in step (b).

3. The method according to claim 1 which comprises

   (a) incubating the faecal bacterial culture in the presence of the tested fiber or substance,
   (b) determining the total amount or total maximum growth rate of the beneficial faecal bacteria populations, and the total amount or total maximum growth rate of the non beneficial faecal bacteria populations,
   (c) subtracting the total amount or total maximum growth rate of the non beneficial faecal bacteria populations from the amount or total maximum growth rate of the beneficial faecal bacteria populations to produce a Prebiotic Index, and
   (d) evaluating or quantifying the effect on the growth and/or modification of the faecal bacterial population as a function of the Prebiotic Index.

4. The method according to claim 3 wherein the identification and quantification of the Prebiotic Index (PI) is defined by the following equation:

$$PI = \mu_{max} B + \mu_{max} L + \mu_{max} E - \mu_{max} Ba - \mu_{max} Cl - \mu_{max} Co - \mu_{max} SRB;$$

wherein PI = prebiotic index; $\mu_{max}$ = maximum growth rates of feacal bacteria in presence of the tested fiber; B = bifidobacteria, L = lactobacilli, E = eubacteria, Ba = bacteroides, Cl = clostridia, Co = coliforms, and SRB = Sulfate Reducing bacteria.

5. The method according to claim 1 which comprises

(a) incubating the faecal bacterial culture in parallel in the presence and in the absence of the tested fiber or substance,
(b) after a certain incubation period, for instance 24 hours, determining the amount or maximum growth rate of the faecal bacteria in the culture in the presence and in the absence of the tested fiber,
(c) subtracting the amount or maximum growth rate of the faecal bacteria in the absence of the tested fiber from the amount or maximum growth rate of the faecal bacteria in the presence of the tested fiber, and
(d) evaluating or quantifying the effect on the growth and/or modification of the faecal bacterial population as a function of the value as determined in step (c).

6. The method according to claim 1 which comprises

(a) incubating the same faecal bacterial culture in the presence and in the absence of the tested fiber,
(b) determining the amount or maximum growth rate of the beneficial faecal bacteria in the presence and in the absence of the tested fiber,
(c) subtracting the amount or maximum growth rate of the beneficial faecal bacteria in the absence of the tested fiber from the amount or maximum growth rate of the beneficial faecal bacteria in the presence of the tested fiber,
(d) determining the amount of the non beneficial faecal bacteria in presence and in the absence of the tested fiber,
(e) subtracting the amount or maximum growth rate of the non beneficial faecal bacteria in the absence of the tested fiber from the amount or maximum growth rate of the non beneficial faecal bacteria in the presence of the tested fiber,
(f) subtracting the value as determined in step (e) from the value as determined in step (c) to produce a Prebiotic Index, and
(g) evaluating or quantifying the effect on the growth and/or modification of the faecal bacterial population as a function of the Prebiotic Index.

7. The method according to any preceding claim wherein the beneficial faecal bacteria is at least one of bifidobacteria, lactobacilli and eubacteria.

8. The method according to any preceding claim wherein the non beneficial faecal bacteria is at least one of bacteroides, clostridia, coliforms and Sulfate Reducing bacteria.

9. The method according to any preceding claim wherein the product resulting from the fermentation of the tested fiber or substance which is quantified is a fermentation end product, for example at least one short chain fatty acid (SCFA), and optionally wherein the quantification of the fermentation end products is calculated through the ratio of lactic acid production over the total SCFA production.

10. The method according to claim 9 wherein the quantification of the fermentation end products is calculated through the ratio of lactic acid production over the production of acetate, butyrate, propionate and lactate.

11. The method according to any preceding claim wherein the maximum assimilation rate of the tested fiber or substance is calculated.

12. The method according to any preceding claim wherein the faecal bacteria population is analyzed by fluorescence *in situ* hybridization.

**13.** A method according to any preceding claim which comprises a step of comparing the prebiotic capability of the tested fiber or substance to the effect of a known prebiotic tested in the same conditions, for example fructooligosaccharide.

**Patentansprüche**

**1.** Verfahren zur Bewertung oder Quantifizierung des präbiotischen Potentials eines Ballaststoffes oder zur Identifizierung einer präbiotischen Substanz, das umfasst:

(a) eine Stufe der Bewertung oder Quantifizierung der Stimulation des Wachstums und/oder der Modifikation einer Fäkalbakterienpopulation durch den getesteten Ballaststoff oder die getestete Substanz,
(b) eine Stufe der Quantifizierung wenigstens eines Produkts, das aus der Fermentation des getesteten Ballaststoffs oder der getesteten Substanz resultiert,
(c) eine Stufe der Quantifizierung der Assimilationsrate des getesteten Ballaststoffs oder der getesteten Substanz,
(d) eine Stufe der Errechnung einer Maßzahl für den präbiotischen Effekt (Measure of the Prebiotic Effect (MPE)) unter Verwendung der folgenden Gleichung:

$$\frac{1}{2}\sqrt{x^2 y^2 + x^2 z^2 + y^2 z^2} = MPE$$

worin x der Wert ist, wie er in Stufe (a) bestimmt wird, y der Wert ist, wie er in Stufe (b) bestimmt wird, und z der Wert ist, wie er in Stufe (c) bestimmt wird, und
(e) eine Stufe der Bewertung oder Quantifizierung des präbiotischen Potentials des getesteten Ballaststoffs oder der getesteten Substanz als eine Funktion des MPE-Werts.

**2.** Verfahren nach Anspruch 1, das umfasst:

(a) Inkubieren einer Fäkalbakterienkultur in Gegenwart des getesteten Ballaststoffs oder der getesteten Substanz, und
(b) Bestimmen der Gesamtmenge und/oder der maximalen Wachstumsrate der nützlichen Fäkalbakterienpopulation, und
(c) Bewerten oder Quantifizieren der Wirkung auf das Wachstum und/oder die Modifikation der Fäkalbakterienpopulation als eine Funktion des Werts, wie er in Stufe (b) bestimmt wird.

**3.** Verfahren nach Anspruch 1, das umfasst:

(a) Inkubieren der Fäkalbakterienkultur in Gegenwart des getesteten Ballaststoffs oder der getesteten Substanz,
(b) Bestimmen der Gesamtmenge oder der gesamten maximalen Wachstumsrate der nützlichen Fäkalbakterienpopulationen, und der Gesamtmenge oder der gesamten maximalen Wachstumsrate der nicht-nützlichen Bakterienpopulationen,
(c) Subtrahieren der Gesamtmenge oder gesamten maximalen Wachstumsrate der nicht-nützlichen Fäkalbakterienpopulationen von der Menge oder der gesamten maximalen Wachstumsrate der nützlichen Fäkalbakterienpopulationen, um einen präbiotischen Index zu erzeugen und
(d) Bewerten oder Quantifizieren der Wirkung auf das Wachstum und/oder auf die Modifikation der Fäkalbakterienpopulation als eine Funktion des präbiotischen Index.

**4.** Verfahren nach Anspruch 3, wobei die Identifikation und Quantifizierung des präbiotischen Index (PI) durch die folgende Gleichung definiert wird:

$$\mathrm{PI} = \mu_{max}B + \mu_{max}L + \mu_{max}E - \mu_{max}Ba - \mu_{max}Cl - \mu_{max}Co - \mu_{max}SRB;$$

worin PI = präbiotische Index; $\mu_{max}$ = die maximalen Wachstumsraten der Fäkalbakterien in Gegenwart des getesteten Ballaststoffs, B = Bifidobakterien, L = Lactobacilli, E = Eubakterien, Ba = Bakteroide, Cl = Clostridien, Co = Coliforme und SRB = Sulfat-reduzierende Bakterien.

5.  Verfahren nach Anspruch 1, das umfasst:

    (a) Inkubieren der Fäkalbakterienkultur parallel in Gegenwart und in Abwesenheit des getesteten Ballaststoffs oder der getesteten Substanz,
    (b) nach einer bestimmten Inkubationszeit, beispielsweise von 24 Stunden, Bestimmen der Menge oder der maximalen Wachstumsrate der Fäkalbakterien in der Kultur in Anwesenheit und in Abwesenheit des getesteten Ballaststoffs,
    (c) Subtrahieren der Menge oder der maximalen Wachstumsrate der Fäkalbakterien in Abwesenheit des getesteten Ballaststoffs von der Menge oder maximalen Wachstumsrate der Fäkalbakterien in Anwesenheit des getesteten Ballaststoffs, und
    (d) Bewerten oder Quantifizieren der Wirkung auf das Wachstum und/oder die Modifikation der Fäkalbakterienpopulation als eine Funktion des Werts, wie er in Stufe (c) bestimmt wird.

6.  Verfahren nach Anspruch 1, das umfasst:

    (a) Inkubieren der gleichen Fäkalbakterienkultur in Anwesenheit und in Abwesenheit des getesteten Ballaststoffs,
    (b) Bestimmen der Menge oder der maximalen Wachstumsrate der nützlichen fäkalen Bakterien in Anwesenheit und in Abwesenheit des getesteten Ballaststoffs,
    (c) Subtrahieren der Menge oder der maximalen Wachstumsrate der nützlichen Fäkalbakterien in Abwesenheit des getesteten Ballaststoffs von der Menge oder der maximalen Wachstumsrate der nützlichen Fäkalbakterien in Gegenwart des getesteten Ballaststoffs,
    (d) Bestimmen der Menge der nicht-nützlichen Fäkalbakterien in Anwesenheit und in Abwesenheit des getesteten Ballaststoffs;
    (e) Subtrahieren der Menge oder der maximalen Wachstumsrate der nicht-nützlichen Fäkalbakterien in Abwesenheit des getesteten Ballaststoffs von der Menge oder der maximalen Wachstumsrate der nicht-nützlichen Fäkalbakterien in Anwesenheit des getesteten Ballaststoffs,
    (f) Subtrahieren des Werts, wie er in Stufe (e) bestimmt wird, von dem Wert, wie er in Stufe (c) bestimmt wird, um einen präbiotischen Index zu erzeugen, und
    (g) Bewerten oder Quantifizieren der Wirkung auf das Wachstum und/oder die Modifikation der Fäkalbakterienpopulation als eine Funktion des präbiotischen Index.

7.  Verfahren nach irgendeinem vorausgehenden Anspruch, wobei die nützliche Fäkalbakterie wenigstens eine ist von Bifidobakterien, Lactobacilli und Eubakterien.

8.  Verfahren nach irgendeinem vorausgehenden Anspruch, wobei die nicht-nützliche Fäkalbakterie wenigstens eine ist von Bakteroiden, Clostridien, Coliformen und Sulfat-reduzierenden Bakterien.

9.  Verfahren nach irgendeinem vorausgehenden Anspruch, wobei das Produkt, das aus der Fermentation des getesteten Ballaststoffs oder der getesteten Substanz resultiert, das quantifiziert wird, ein Fermentations-Endprodukt ist, beispielsweise wenigstens eine kurzkettige Fettsäure (SCFA), und wobei ggf. die Quantifizierung der Fermentations-Endprodukte errechnet wird anhand des Verhältnisses der Milchsäureproduktion, bezogen auf die gesamte SCFA-Produktion.

10. Verfahren nach Anspruch 10, wobei die Quantifizierung des Fermentationsendprodukts errechnet wird anhand des Verhältnisses der Milchsäureproduktion bezogen auf die Produktion von Acetat, Butyrat, Propionat und Lactat.

11. Verfahren nach irgendeinem vorausgehenden Anspruch, wobei die maximale Assimilationsrate des getesteten Ballaststoffs oder der getesteten Substanz errechnet wird.

12. Verfahren nach irgendeinem vorausgehenden Anspruch, wobei die Fäkalbakterienpopulation mittels Fluoreszenz-in situ-Hybridisierung analysiert wird.

13. Verfahren nach irgendeinem vorausgehenden Anspruch, das die Stufe des Vergleichs des präbiotischen Potentials des getesteten Ballaststoffs oder der getesteten Substanz mit der Wirkung eines bekannten Präbiotikums umfasst, das unter den gleichen Bedingungen getestet wird, beipielsweise von Fructooligosaccharid.

**Revendications**

1.  Méthode pour évaluer ou quantifier la capacité prébiotique d'une fibre ou pour identifier une substance prébiotique, qui comprend

    (a) une étape d'évaluation ou de quantification de la stimulation par la fibre ou substance testée de la croissance et/ou de la modification d'une population bactérienne fécale,
    (b) une étape de quantification d'au moins un produit résultant de la fermentation de la fibre ou substance testée,
    (c) une étape de quantification de la vitesse d'assimilation de la fibre ou substance testée,
    (d) une étape de calcul de la mesure de l'effet prébiotique (MPE) en utilisant l'équation suivante :

$$\frac{1}{2}\sqrt{x^2 y^2 + x^2 z^2 + y^2 z^2} = MPE$$

    dans laquelle x représente la valeur telle que déterminée dans l'étape (a), y représente la valeur telle que déterminée dans l'étape (b) et z représente la valeur telle que déterminée dans l'étape (c), et
    (e) une étape d'évaluation ou de quantification de la capacité prébiotique de la fibre ou substance testée en fonction de la MPE.

2.  Méthode suivant la revendication 1 qui comprend

    (a) la mise en incubation de la culture bactérienne fécale en présence de la fibre ou substance testée, et
    (b) la détermination de la quantité totale et/ou de la vitesse maximale de croissance de la population bactérienne fécale bénéfique, et
    (c) l'évaluation ou la quantification de l'effet sur la croissance et/ou la modification de la population bactérienne fécale en fonction de la valeur telle que déterminée dans l'étape (b).

3.  Méthode suivant la revendication 1 qui comprend

    (a) la mise en incubation de la culture bactérienne fécale en présence de la fibre ou substance testée,
    (b) la détermination de la quantité totale ou de la vitesse de croissance maximale totale des populations bactériennes fécales bénéfiques et de la quantité totale ou vitesse de croissance maximale totale des populations bactériennes fécales non bénéfiques,
    (c) la soustraction de la quantité totale ou de la vitesse de croissance maximale totale des populations bactériennes fécales non bénéfiques de la quantité ou de la vitesse de croissance maximale totale des populations bactériennes fécales bénéfiques pour produire un indice prébiotique, et
    (d) l'évaluation ou la quantification de l'effet sur la croissance et/ou modification de la population bactérienne fécale en fonction de l'indice prébiotique.

4.  Méthode suivant la revendication 3, dans laquelle l'identification et la quantification de l'indice prébiotique (PI) sont définies par l'équation suivante :

```
PI = µmax B + µmax L + µmax E - µmax Ba - µmax Cl -
µmax Co - µmax SRB ;
```

    dans laquelle PI = indice prébiotique ; $\mu$max = vitesses maximales de croissance des bactéries fécales en présence de la fibre testée ; B = bifidobactéries ; L = lactobacilles ; E = eubactéries ; Ba = Bacteroïdes ; Cl = Clostridia ; Co = coliformes ; et SRB = bactéries sulfato-réductrices.

5.  Méthode suivant la revendication 1, qui comprend

(a) la mise en incubation de la culture bactérienne fécale en parallèle en la présence et en l'absence de la fibre ou substance testée,

(b) après une certaine période d'incubation, par exemple de 24 heures, la détermination de la quantité ou de la vitesse maximale de croissance des bactéries fécales dans la culture en la présence et en l'absence de la fibre testée,

(c) la soustraction de la quantité ou de la vitesse maximale de croissance des bactéries fécales en l'absence de la fibre testée de la quantité ou vitesse maximale de croissance des bactéries fécales en présence de la fibre testée, et

(d) l'évaluation ou la quantification de l'effet sur la croissance et/ou la modification de la population bactérienne fécale en fonction de la valeur telle que déterminée dans l'étape (c).

6. Méthode suivant la revendication 1, qui comprend

(a) la mise en incubation de la même culture bactérienne fécale en la présence et en l'absence de la fibre ou substance testée,

(b) la détermination de la quantité ou de la vitesse maximale de croissance des bactéries fécales bénéfiques en la présence et en l'absence de la fibre testée,

(c) la soustraction de la quantité ou de la vitesse maximale de croissance des bactéries fécales bénéfiques en l'absence de la fibre testée de la quantité ou de la vitesse maximale de croissance des bactéries fécales bénéfiques en présence de la fibre testée,

(d) la détermination de la quantité des bactéries fécales non bénéfiques en la présence et en l'absence de la fibre testée,

(e) la soustraction de la quantité ou de la vitesse maximale de croissance des bactéries fécales non bénéfiques en l'absence de la fibre testée de la quantité ou de la vitesse maximale de croissance des bactéries fécales non bénéfiques en présence de la fibre testée,

(f) la soustraction de la valeur telle que déterminée dans l'étape (e) de la valeur telle que déterminée dans l'étape (c) pour produire un indice prébiotique et

(g) l'évaluation ou la quantification de l'effet sur la croissance et/ou la modification de la population bactérienne fécale en fonction de l'indice prébiotique.

7. Méthode suivant l'une quelconque des revendications précédentes, dans laquelle les bactéries fécales bénéfiques consistent en au moins un type des bactéries consistant en bifidobactéries, lactobacilles et eubactéries.

8. Méthode suivant l'une quelconque des revendications précédentes, dans laquelle les bactéries fécales non bénéfiques consistent en au moins un type de bactéries consistant en Bacteroïdes, Clostridia, coliformes et bactéries sulfato-réductrices.

9. Méthode suivant l'une quelconque des revendications précédentes, dans laquelle le produit résultant de la fermentation de la fibre ou substance testée qui est quantifié est un produit final de fermentation, par exemple au moins un acide gras à chaîne courte (SCFA), et facultativement dans laquelle la quantification des produits finals de la fermentation est calculée par le rapport de la production d'acide lactique à la production totale de SCFA.

10. Méthode suivant la revendication 9, dans laquelle la quantification des produits finals de fermentation est calculée par le rapport de la production d'acide lactique à la production d'acétate, de butyrate, de propionate et de lactate.

11. Méthode suivant l'une quelconque des revendications précédentes, dans laquelle la vitesse maximale d'assimilation de la fibre ou substance testée est calculée.

12. Méthode suivant l'une quelconque des revendications précédentes, dans laquelle la population bactérienne fécale est analysée par hybridation in situ par fluorescence.

13. Méthode suivant l'une quelconque des revendications précédentes, qui comprend une étape de comparaison de la capacité prébiotique de la fibre ou substance testée avec l'effet d'un prébiotique connu testé dans les mêmes conditions, par exemple un fructo-oligosaccharide.

Figure 1/4:

Figure 2/4:

Figure 3/4:

Figure 4/4:

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5260279 A

**Non-patent literature cited in the description**

- **R. PALFRAMAN et al.** *Letters in Applied Microbiology,* 2003, vol. 37, 281-284

- **E. OLANO-MARTIN et al.** *Journal of Applied Microbiology,* 2002, vol. 93, 505-511